# FASCICULE DE BREVET EUROPEEN

(11) **EP 2 809 342 B1**
(45) Date de publication et mention de la délivrance du brevet: **21.12.2016**
(21) Numéro de dépôt: 13712827.8
(22) Date de dépôt: 31.01.2013
(51) Int. Cl.: C07K 14/81, C07K 5/083, C07K 5/11, C07K 14/78, A61K 38/00, A61K 8/64, A61Q 19/08

(54) **PEPTIDE BIFONCTIONNEL**
BIFUNKTIONELLES PEPTID
BIFUNCTIONAL PEPTIDE

(30) Priorité: 01.02.2012 FR 1250932
(43) Date de publication de la demande: 10.12.2014
(73) Titulaire: Regentis International, 51100 Reims (FR); Université De Reims Champagne-Ardenne, 51100 Reims (FR); Centre National de la Recherche Scientifique, 75794 Paris Cedex 16 (FR)
(72) Inventeur: HORNEBECK, William, F-51100 Reims (FR); ATTIA, Joan, F-51100 Reims (FR); LORIMIER, Sandrine, F-51100 Reims (FR); ANTONICELLI, Frank, F-51420 Witry-les-Reims (FR)
(74) Mandataire: Rhein, Alain
(86) Numéro de dépôt international: PCT/FR2013/000033
(87) Numéro de publication internationale: WO 2013/114013

(56) Documents cités:
- EP-A1- 1 275 372
- WO-A2-03/101376
- DE-A1-102004 055 541
- ALIX A J: "A turning point in the knowledge of the structure-function-activity relations of elastin", JOURNAL DE LA SOCIETE DE BIOLOGIE, SOCIETE DE BIOLOGIE, PARIS, FR, vol. 195, no. 2, 1 janvier 2001 (2001-01-01), pages 181-193, XP009105680, ISSN: 1295-0661 cité dans la demande

### LISTE DE SEQUENCES

<110> REGENTIS INTERNATIONAL
   UNIVERSITE DE REIMS CHAMPAGNE-ARDENNE
   CENTRE NATIONAL DE LA RECHERCHE SCIENTIFIQUE
<120> PEPTIDE BIFONCTIONNEL
<130> 9R59 BT FR 1
<160> 26
<210> 1
   <211> 25
   <212> PRT
   <213> Séquence artificielle
   <220>
   <223> Peptide bifonctionnel apte à activer la synthèse de collagène et à inhiber la production de métallo-protéinases matricielles
<400> 1 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Ile-Leu
   15                  20                  25
<210> 2
   <211> 25
   <212> PRT
   <213> Séquence artificielle
   <220>
   <223> Peptide bifonctionnel apte à activer la synthèse de collagène et à inhiber la production de métallo-protéinases matricielles
<400> 2 
Val-Gly-Thr-Ala-Pro-Gly-Val-Gly-Thr-Ala-Pro-Gly-Val-
 1               5                   10

Gly-Thr-Ala-Pro-Gly-Arg-Val-Arg-Leu-Gly-Ile-Leu
    15                  20                  25
<210> 3
   <211> 25
   <212> PRT
   <213> Séquence artificielle
   <220>
   <223> Peptide bifonctionnel apte à activer la synthèse de collagène et à inhiber la production de métallo-protéinases matricielles
<400> 3 
Val-Gly-Gln-Ala-Pro-Gly-Val-Gly-Gln-Ala-Pro-Gly-Val-
 1               5                   10

Gly-Gln-Ala-Pro-Gly-Arg-Val-Arg-Leu-Gly-Ile-Leu
    15                  20                  25
<210> 4
   <211> 25
   <212> PRT
   <213> Séquence artificielle
   <220>
   <223> Peptide bifonctionnel apte à activer la synthèse de collagène et à inhiber la production de métallo-protéinases matricielles
<400> 4 
Val-Gly-Ala-Ala-Pro-Gly-Val-Gly-Ala-Ala-Pro-Gly-Val-
 1              5                   10

Gly-Ala-Ala-Pro-Gly-Arg-Val-Arg-Leu-Gly-Ile-Leu
    15                  20                  25
<210> 5
   <211> 25
   <212> PRT
   <213> Séquence artificielle
   <220>
   <223> Peptide bifonctionnel apte à activer la synthèse de collagène et à inhiber la production de métallo-protéinases matricielles
<400> 5 
Val-Gly-Leu-Ala-Pro-Gly-Val-Gly-Leu-Ala-Pro-Gly-Val-
 1                 5                   10

Gly-Leu-Ala-Pro-Gly-Arg-Val-Arg-Leu-Gly-Ile-Leu
    15                  20                  25
<210> 6
   <211> 25
   <212> PRT
   <213> Séquence artificielle
   <220>
   <223> Peptide bifonctionnel apte à activer la synthèse de collagène et à inhiber la production de métallo-protéinases matricielles
<400> 6 
Val-Gly-Val-Leu-Pro-Gly-Val-Gly-Val-Leu-Pro-Gly-Val-
 1               5                   10

Gly-Val-Leu-Pro-Gly-Arg-Val-Arg-Leu-Gly-Ile-Leu
    15                  20                  25
<210> 7
   <211> 25
   <212> PRT
   <213> Séquence artificielle
   <220>
   <223> Peptide bifonctionnel apte à activer la synthèse de collagène et à inhiber la production de métallo-protéinases matricielles
<400> 7 
Val-Gly-Val-Ile-Pro-Gly-Val-Gly-Val-Ile-Pro-Gly-Val-
1               5                   10

Gly-Val-Ile-Pro-Gly-Arg-Val-Arg-Leu-Gly-Ile-Leu
    15                  20                  25
<210> 8
   <211> 25
   <212> PRT
   <213> Séquence artificielle
   <220>
   <223> Peptide bifonctionnel apte à activer la synthèse de collagène et à inhiber la production de métallo-protéinases matricielles
<400> 8 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Ile-Ile-Leu
    15                  20                  25
<210> 9
   <211> 25
   <212> PRT
   <213> Séquence artificielle
   <220>
   <223> Peptide bifonctionnel apte à activer la synthèse de collagène et à inhiber la production de métallo-protéinases matricielles
<400> 9 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Leu-Ile-Leu
    15                  20                  25
<210> 10
   <211> 25
   <212> PRT
   <213> Séquence artificielle
   <220>
   <223> Peptide bifonctionnel apte à activer la synthèse de collagène et à inhiber la production de métallo-protéinases matricielles
<400> 10 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Val-Leu
   15                  20                  25
<210> 11
   <211> 25
   <212> PRT
   <213> Séquence artificielle
   <220>
   <223> Peptide bifonctionnel apte à activer la synthèse de collagène et à inhiber la production de métallo-protéinases matricielles
<400> 11 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Ala-Leu
    15                  20                  25
<210> 12
   <211> 25
   <212> PRT
   <213> Séquence artificielle
   <220>
   <223> Peptide bifonctionnel apte à activer la synthèse de collagène et à inhiber la production de métallo-protéinases matricielles
<400> 12 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Gly-Leu
    15                  20                  25
<210> 13
   <211> 25
   <212> PRT
   <213> Séquence artificielle
   <220>
   <223> Peptide bifonctionnel apte à activer la synthèse de collagène et à inhiber la production de métallo-protéinases matricielles
<400> 13 
Val-Gly-Val-Ala-Pro-Gly-Val-Gly-Val-Ala-Pro-Gly-Val-
 1               5                   10
 
Gly-Val-Ala-Pro-Gly-Arg-Val-Arg-Leu-Gly-Ile-Phe
15                  20                  25
<210> 14
   <211> 25
   <212> PRT
   <213> Séquence artificielle
   <220>
   <223> Peptide bifonctionnel apte à activer la synthèse de collagène et à inhiber la production de métallo-protéinases matricielles
<400> 14 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Ile-Ala
    15                  20                  25
<210> 15
   <211> 25
   <212> PRT
   <213> Séquence artificielle
   <220>
   <223> Peptide bifonctionnel apte à activer la synthèse de collagène et à inhiber la production de métallo-protéinases matricielles
<400> 15 
Val-Gly-Val-Ala-Pro-Gly-Val-Gly-Val-Ala-Pro-Gly-Val-
 1               5                   10

Gly-Val-Ala-Pro-Gly-Arg-Val-Arg-Leu-Gly-Ile-Ile
    15                  20                  25
<210> 16
   <211> 25
   <212> PRT
   <213> Séquence artificielle
   <220>
   <223> Peptide bifonctionnel apte à activer la synthèse de collagène et à inhiber la production de métallo-protéinases matricielles
<400> 16 
Val-Gly-Val-Ala-Pro-Gly-Val-Gly-Val-Ala-Pro-Gly-Val-
1               5                   10

Gly-Val-Ala-Pro-Gly-Arg-Val-Arg-Leu-Gly-Ile-Val
    15                  20                  25
<210> 17
   <211> 25
   <212> PRT
   <213> Séquence artificielle
   <220>
   <223> Peptide bifonctionnel apte à activer la synthèse de collagène et à inhiber la production de métallo-protéinases matricielles
<400> 17 
Val-Gly-Val-Ala-Pro-Gly-Val-Gly-Val-Ala-Pro-Gly-Val-
 1               5                   10

Gly-Val-Ala-Pro-Gly-Arg-Ser-Arg-Leu-Gly-Ile-Leu
    15                  20                  25
<210> 18
   <211> 25
   <212> PRT
   <213> Séquence artificielle
   <220>
   <223> Peptide bifonctionnel apte à activer la synthèse de collagène et à inhiber la production de métallo-protéinases matricielles
<400> 18 
Val-Gly-Val-Ala-Pro-Gly-Val-Gly-Val-Ala-Pro-Gly-Val-
 1               5                   10

Gly-Val-Ala-Pro-Gly-Arg-Tyr-Arg-Leu-Gly-Ile-Leu
    15                  20                  25
<210> 19
   <211> 25
   <212> PRT
   <213> Séquence artificielle
<220>
   <223> Peptide bifonctionnel apte à activer la synthèse de collagène et à inhiber la production de métallo-protéinases matricielles
<400> 19 
Val-Gly-Val-Ala-Pro-Gly-Val-Gly-Val-Ala-Pro-Gly-Val-
 1               5                   10

Gly-Val-Ala-Pro-Gly-Arg-Gly-Arg-Leu-Gly-Ile-Leu
    15                  20                  25
<210> 20
   <211> 25
   <212> PRT
   <213> Séquence artificielle
   <220>
   <223> Peptide bifonctionnel apte à activer la synthèse de collagène et à inhiber la production de métallo-protéinases matricielles
<400> 20 
Val-Gly-Val-Ala-Pro-Gly-Val-Gly-Val-Ala-Pro-Gly-Val-
 1               5                   10

Gly-Val-Ala-Pro-Gly-Arg-Ala-Arg-Leu-Gly-Ile-Leu
    15                  20                  25
<210> 21
   <211> 25
   <212> PRT
   <213> Séquence artificielle
   <220>
   <223> Peptide bifonctionnel apte à activer la synthèse de collagène et à inhiber la production de métallo-protéinases matricielles
<400> 21 
Val-Gly-Val-Ala-Pro-Gly-Val-Gly-Val-Ala-Pro-Gly-Val-
 1               5                   10

Gly-Val-Ala-Pro-Gly-Arg-Arg-Arg-Leu-Gly-Ile-Leu
    15                  20                  25
<210> 22
   <211> 25
   <212> PRT
   <213> Séquence artificielle
   <220>
   <223> Peptide bifonctionnel apte à activer la synthèse de collagène et à inhiber la production de métallo-protéinases matricielles
<400> 22 
Val-Gly-Val-Ala-Pro-Gly-Val-Gly-Val-Ala-Pro-Gly-Val-
 1               5                   10

Gly-Val-Ala-Pro-Gly-Arg-Val-Arg-Ser-Gly-Ile-Leu
    15                  20                  25
<210> 23
   <211> 25
   <212> PRT
   <213> Séquence artificielle
   <220>
   <223> Peptide bifonctionnel apte à activer la synthèse de collagène et à inhiber la production de métallo-protéinases matricielles
<400> 23 
Val-Gly-Val-Ala-Pro-Gly-Val-Gly-Val-Ala-Pro-Gly-Val-
 1               5                   10

Gly-Val-Ala-Pro-Gly-Arg-Val-Arg-Tyr-Gly-Ile-Leu
    15                  20                  25
<210> 24
   <211> 25
   <212> PRT
   <213> Séquence artificielle
   <220>
   <223> Peptide bifonctionnel apte à activer la synthèse de collagène et à inhiber la production de métallo-protéinases matricielles
<400> 24 
Val-Gly-Val-Ala-Pro-Gly-Val-Gly-Val-Ala-Pro-Gly-Val-
 1               5                   10

Gly-Val-Ala-Pro-Gly-Arg-Val-Arg-Gly-Gly-Ile-Leu
    15                  20                  25
<210> 25
   <211> 25
   <212> PRT
   <213> Séquence artificielle
   <220>
   <223> Peptide bifonctionnel apte à activer la synthèse de collagène et à inhiber la production de métallo-protéinases matricielles
<400> 25 
Val-Gly-Val-Ala-Pro-Gly-Val-Gly-Val-Ala-Pro-Gly-Val-
 1               5                   10

Gly-Val-Ala-Pro-Gly-Arg-Val-Arg-Ala-Gly-Ile-Leu
    15                  20                  25
<210> 26
   <211> 25
   <212> PRT
   <213> Séquence artificielle
   <220>
   <223> Peptide bifonctionnel apte à activer la synthèse de collagène et à inhiber la production de métallo-protéinases matricielles
<400> 26

Val-Gly-Val-Ala-Pro-Gly-Val-Gly-Val-Ala-Pro-Gly-Val-
 1               5                   10

Gly-Val-Ala-Pro-Gly-Arg-Val-Arg-Arg-Gly-Ile-Leu
    15                  20                  25

## Description

La présente invention concerne un peptide qui trouvera son application notamment dans les domaines cosmétiques et pharmaceutiques.

La présente invention trouvera son application principalement dans le domaine de la réparation ou de la régénération du tissu dermique atteint notamment par le vieillissement cutané.

Le vieillissement cutané est du à un déséquilibre plus ou moins important des mécanismes de contrôle de la synthèse et de la dégradation des composants de la matrice extracellulaire du derme, ce dernier constituant la couche la plus interne de la peau. Ainsi, lors du vieillissement cutané, on observe, au sein de la matrice extracellulaire du derme, d'une part, une diminution de la synthèse de certaines macromolécules, notamment le collagène et l'élastine, et d'autre part, une augmentation de l'expression de certaines enzymes comme les Métallo-Protéinases Matricielles, ou MMP. Ces dernières présentent notamment une activité collagénolytique et élastolytique, et contribuent de ce fait à la dégradation des macromolécules de la matrice du derme. En conséquence, le derme perd son tonus, la peau se distend, ce qui aboutit à l'apparition de rides. Ce phénomène peut également être amplifié par des facteurs extérieurs, tels qu'une exposition aux ultra-violets, à la pollution, au stress, ou encore au tabac.

Traditionnellement, il est connu de l'état de la technique des stratégies destinées à ralentir l'apparition de rides. De telles stratégies consistent notamment en :
- une desquamation, ou dermabrasion, qui correspond à un traitement agressif de la peau dans le but de réduire l'épaisseur de cette dernière ;
- les injections antirides d'acide hyaluronique ou de toxine botulique qui présentent respectivement un effet de comblement de rides et une paralysie musculaire ciblée atténuant temporairement les rides ; ces techniques sont cependant onéreuses et montrent des effets uniquement à court terme ;

- l'application de traitement superficiels, tels que des produits hydratants, des crèmes anti-rides ou lissantes, qui ralentissent l'apparition des rides mais sans corriger celles qui sont déjà formées ;
- des thérapies à base de cellules souches autologues qui sont encore peu maitrisées.

Depuis quelques années, d'autres stratégies sont mises en place par les laboratoires dans le but de lutter contre les effets du vieillissement cutané.

En particulier, l'utilisation de composés chimiques, tels que des dérivés N-acylaminoamides, est proposée dans la demande de brevet européen EP 1 275 372. Plus particulièrement, ce document décrit une composition dans laquelle est incorporée, outre le dérivé N-acylaminoamide susmentionné, une protéine inhibitrice des métalloprotéinases, ces dernières étant impliquées dans la dégradation du collagène.

Cependant, une telle solution n'est pas optimale, en particulier car elle nécessite, d'une part, une combinaison de plusieurs composés et que, d'autre part, elle ne permet pas d'agir positivement sur la synthèse de collagène.

L'utilisation de certains dérivés peptidiques est également développée pour prévenir notamment les symptômes du vieillissement cutané.

De ce fait, on connait par exemple, dans le document de brevet FR 285489, des compositions cosmétiques comportant des dérivés peptidiques, ces derniers provenant de la protéine élastine. Plus particulièrement, le dérivé peptidique peut présenter la séquence suivante R1-(AA)n-Val-Gly-Val-Ala-Pro-Gly-OR2, où
- R1 correspond à H ou à une chaine alcoyle comprenant entre 2 et 22 carbones,
- (AA)n correspond à une chaine peptidique avec AA consistant en un acide aminé quelconque ou un dérivé d'acide aminé et ou n est compris entre 0 et 3,
- R2 correspond à H ou à une chaine alkyle comprenant entre 1 et 24 carbones.

Une composition cosmétique incorporant un tel dérivé peptidique permet notamment un effet raffermissant et restructurant.

Cependant, un tel dérivé peptidique ou une composition incorporant ce dérivé ne permet pas d'inhiber l'activité et/ou l'expression des protéinases MMP qui sont impliquées dans la dégradation des collagènes, ces derniers constituant les principales protéines de la matrice extracellulaire des tissus de l'organisme, et notamment du derme. Le collagène permet notamment de conférer aux tissus leur résistance aux forces de tension. Ainsi, le peptide décrit dans le document susmentionné ne permet pas d'éviter la dégradation de cette protéine essentielle pour préserver le tonus dermique.

On connait aussi, par le document de brevet WO 2006053688 une combinaison incorporant plusieurs peptides et utilisée dans le traitement antirides. En particulier, les peptides de cette combinaison interviennent à plusieurs niveaux du processus de la synthèse du collagène et/ou de la fibronectine, notamment en stimulant cette synthèse.

Le document de brevet DE 10 2004 055 541 décrit quant à lui une composition comportant une combinaison de peptides dans l'optique de stimuler la libération du facteur de croissance TGF-β, ce dernier pouvant induire la synthèse de collagène.

Cependant, les peptides décrits dans ces documents de brevet présentent les mêmes inconvénients que ceux cités précédemment, et notamment le fait que la séquence proposée ici ne permet pas de diminuer l'expression des enzymes responsables de la dégradation des protéines de la matrice extracellulaire, et en particulier du collagène.

L'invention offre la possibilité de pallier les divers inconvénients de l'état de la technique en proposant un peptide bifonctionnel présentant à la fois la propriété de stimuler la synthèse des collagènes et celle d'inhiber les protéinases entrant dans la cascade de dégradation de ces derniers.

A cet effet, la présente invention concerne un peptide bifonctionnel apte à activer la synthèse de collagène et à inhiber la production de métallo-protéinases matricielles, ledit peptide présentant une séquence comportant trois parties peptidiques A, B et C,
- la première partie peptidique A correspondant à un hexapeptide répété au moins trois fois, ladite partie A étant apte à se lier à une protéine récepteur liant l'élastine pour stimuler la synthèse de collagène,
- la deuxième partie peptidique B correspondant à un tétrapeptide apte à agir en tant qu'inhibiteur compétitif de la protéase urokinase et à être clivé par ladite protéase, et,
- la troisième partie peptidique C correspondant à un tripeptide occupant au moins un site actif des métallo-protéinases matricielles pour permettre une inhibition desdites protéinases.

La première partie peptidique A stimulant la synthèse de collagène présente la séquence X1-Gly-X2-X3-Pro-Gly, cette séquence étant répétée au moins trois fois, avec :
- X1 correspondant à un acide aminé quelconque,
- X2 correspondant à un acide aminé choisi parmi Val, Thr, Gln, Ala, Leu,
- X3 correspondant à un acide aminé choisi parmi Ala, Leu, Ile.

Avantageusement, la première partie peptidique A dudit peptide présente la séquence Val-Gly-Val-Ala-Pro-Gly.

La deuxième partie peptidique B, clivable par une protéase, présente la séquence Arg-Y1-Arg-Y2, avec Y1 et Y2 correspondant chacun à un acide aminé choisi parmi Ser, Tyr, Gly, Ala, Arg, Val, Leu.

Préférentiellement la deuxième partie peptidique B dudit peptide présente la séquence Arg-Val-Arg-Leu.

La troisième partie C, permettant une inhibition des métallo-protéinases matricielles, présente la séquence Z1-Ile-Z2, avec :
- Z1 correspondant à un acide aminé choisi parmi Gly, Ile, Leu et
- Z2 correspondant à un acide aminé choisi parmi Leu, Phe, Ala, Ile, Val.

De façon avantageuse, la troisième partie peptidique C dudit peptide présente la séquence Gly-Ile-Leu.

Selon un exemple de réalisation préférentiel, le peptide selon l'invention présente la séquence S1 suivante, correspondant à la séquence SEQ ID n°1 :
S1: N-(Val-Gly-Val-Ala-Pro-Gly)n-Arg-Val-Arg-Leu-Gly-Ile-Leu-OH
où N et OH correspondent respectivement aux extrémités N-terminale et C-terminale dudit peptide
et où n=3

Cependant, la séquence du peptide bifonctionnel selon l'invention peut également correspondre à l'une des séquences SEQ ID n°2 à SEQ ID n°26.

Préférentiellement, le peptide bifonctionnel selon l'invention est obtenu par synthèse chimique. De plus, le peptide peut être avantageusement conservé sous forme lyophilisée.

De façon avantageuse, le peptide bifonctionnel selon l'invention peut être utilisé pour le traitement des maladies de cicatrisation chroniques, notamment le traitement des escarres ou des ulcères.

Le peptide bifonctionnel selon l'invention peut également être utilisé pour la réparation et/ou la régénération du tissu dermique, notamment pour le traitement du vieillissement cutané.

L'invention concerne également une composition cosmétique et/ou pharmaceutique incorporant le peptide bifonctionnel selon l'invention.

Préférentiellement, la concentration en peptide bifonctionnel dans la composition cosmétique est comprise entre 10µg/mL et 1mg/mL, et de préférence sensiblement égale à 100µg/mL.

Le peptide bifonctionnel selon la présente invention comporte de nombreux avantages. D'une part, il permet une stimulation de la synthèse de protéines de collagène, et notamment des collagènes de type I et de type III. L'augmentation de production de ce dernier type de collagène est particulièrement intéressante ; en effet, ce type de collagène est appelé collagène de type « foetal » car il est principalement synthétisé au stade foetus et pendant l'enfance. Ce type de collagène permet une cicatrisation particulièrement optimale des tissus, appelée cicatrisation « parfaite ». D'autre part, ledit peptide permet de diminuer la collagénolyse par des protéases. Par ailleurs, la stimulation de la synthèse de collagène et l'inhibition des métalloprotéinases sont obtenues simultanément grâce aux parties peptidiques A et C respectivement du peptide bifonctionnel, celui-ci comportant en outre une partie peptidique B reliant les deux autres parties et étant apte à être clivée par l'urokinase. De ce fait, le peptide selon l'invention peut présenter une courte séquence, de l'ordre de 25 acides aminés, ce qui facilite sa synthèse par voie chimique.

D'autres caractéristiques et avantages de l'invention ressortiront de la description détaillée qui va suivre des modes de réalisation non limitatifs de l'invention, en référence aux figures annexées dans lesquelles :
- la figure 1 est une représentation schématique du mécanisme d'action du peptide selon l'invention.
- la figure 2 correspond à une représentation schématique de la cascade enzymatique du système plasminogène/plasmine.
- les figures 3A et 3B correspondent à des histogrammes illustrant respectivement la synthèse de collagène de type I et de type III par des fibroblastes traités par le peptide selon l'invention à une concentration de 100µg/ml ou par des fragments de ce même peptide.
- la figure 4 montre sous forme d'histogrammes l'expression génique des gènes COL1A1 et COL3A1 codant respectivement pour les collagènes de type I et III dans des cultures de fibroblastes dermiques traités par le peptide selon l'invention ou par des fragments de ce même peptide.
- la figure 5 illustre, en fonction de l'âge des patients (35, 42 et 53 ans), l'effet du peptide bifonctionnel selon l'invention sur l'expression protéique des collagènes de type I et III par les fibroblastes dermiques issus respectivement de ces patients.
- les figures 6A et 6B illustrent l'effet du peptide bifonctionnel sur l'activité de l'urokinase par rapport à un substrat synthétique.
- la figure 7A et 7B illustrent l'effet du peptide bifonctionnel sur l'activité de la MMP-1 par rapport à un substrat synthétique.
- les figures 8A et 8B représentent le profil d'élution du peptide selon l'invention respectivement sans traitement et avec traitement par l'urokinase.

A titre préliminaire et informatif, la présente description utilise le code international à trois lettres pour désigner les acides aminés. Ainsi Ala correspond à l'alanine (A), Cys à la cystéine (C), Asp à l'acide aspartique (D), Glu à l'acide glutamique (E), Phe à la phénylalanine (F), Gly à la glycine (G), His à l'histidine (H), Ile à l'isoleucine (I), Lys à la lysine (K), Leu à la leucine (L), Met à la méthionine (M), Asn à l'asparagine (N), Pro à la proline (P), Gln à la glutamine (Q), Arg à l'arginine (R), Ser à la sérine (S), Thr à la thréonine (T), Val à la valine (V), Trp au tryptophane (W) et Tyr à la tyrosine (Y).

Le vieillissement cutané et l'apparition de rides qui en résulte sont caractérisés, au niveau cellulaire et moléculaire, par une diminution de l'expression des gènes responsables de la synthèse des macromolécules entrant dans la composition de la matrice extracellulaire, telles que les collagènes ou l'élastine. De plus, la baisse de la production de ces protéines matricielles est accompagnée généralement d'une surexpression des enzymes qui sont responsables de la dégradation desdites protéines matricielles ; ces enzymes, les métalloprotéinases matricielles ou MMP, sont donc en partie à l'origine de la baisse de tonus au niveau du derme, de la distension de la peau et, à terme, de l'apparition de rides.

Les protéines de collagène sont les protéines prépondérantes dans la constitution de la matrice extracellulaire des tissus de l'organisme. En particulier, les collagènes sont présents à hauteur de 90% au niveau du derme, ce dernier constituant le support solide de la peau et jouant un rôle à la fois nutritif, thermorégulateur et de défense contre les micro-organismes pathogènes. Le derme est principalement constitué de cellules appelées fibroblastes, qui participent à la synthèse notamment des collagènes, ainsi qu'à l'organisation de la matrice extracellulaire.

Ainsi, la charpente de la matrice extracellulaire est constituée de collagène, mais également d'élastine, de glycoprotéines structurales et de protéoglycanes. Plus particulièrement, la matrice dermique comporte des fibrilles de collagène de type I et III et d'un coeur de collagène de type V.

Ces derniers sont susceptibles d'être dégradés par des protéines appelées MMP, dont la famille comporte pas moins de 26 membres. Plus particulièrement, ces protéines correspondent à des endopeptidases, c'est-à-dire qui rompent les liaisons peptidiques à l'intérieur de la protéine.

Les travaux menés par des scientifiques (Voorhes et al., Hornebeck et al., 2009) ont permis de mettre en évidence que la sénescence des cellules dermiques, les fibroblastes, est caractérisée par une augmentation de l'expression de ces endopeptidases, notamment en ce qui concerne la MMP-1. Cette dernière appartient au groupe des collagénases interstitielles, et est responsable du clivage des protéines de collagène de type I et III. La MMP-1 est considérée comme la principale collagénase impliquée au cours du vieillissement cutané, que celui-ci soit chronologique ou photoinduit.

Ainsi, la diminution des protéines matricielles dermiques, et notamment les collagènes, associée à une augmentation des enzymes de dégradation, notamment les MMP-1, sont des éléments importants intervenant dans le vieillissement cutané qui se traduit par une diminution du tonus de la peau et une apparition de rides visibles.

C'est donc dans le cadre d'une démarche inventive que les inventeurs ont développé un peptide présentant plusieurs fonctions et permettant à la fois d'agir au niveau des processus de synthèse des molécules de collagène ainsi qu'à différents niveau des cascades de dégradation de ces mêmes molécules.

Ainsi, de façon tout particulièrement avantageuse, le peptide bifonctionnel selon la présente invention présente la particularité de favoriser le rétablissement d'un équilibre entre les processus anaboliques (synthèse) et cataboliques (dégradation) de la matrice extracellulaire notamment dermique.

De façon particulièrement préférentielle, le peptide selon l'invention est bifonctionnel et comporte une séquence qui peut être divisée en trois parties :
- une première partie peptidique, appelée partie A, qui présente avantageusement la capacité d'augmenter la synthèse de collagène ;
- une deuxième partie peptidique, appelée partie B, qui présente préférentiellement une séquence reconnue par une protéase, l'urokinase, ladite partie peptidique B pouvant être alors clivée par cette protéase ;
- une troisième partie peptidique, appelée partie C, cette dernière étant apte à inhiber les MMP.

Le peptide selon la présente invention permet donc, d'une part, de stimuler la synthèse de collagène et, d'autre part, de réduire la dégradation dudit collagène en inhibant l'urokinase et les protéases MMP, notamment la MMP-1.

Plus particulièrement, la partie peptidique A, permettant une augmentation de la synthèse de collagène, consiste en un hexapeptide, c'est-à-dire une succession de 6 résidus d'acides aminés. Avantageusement, cet hexapeptide est répété au moins 3 fois au sein du peptide bifonctionnel selon la présente invention.

Préférentiellement, la partie peptidique A du peptide bifonctionnel présente la séquence suivante X1-Gly-X2-X3-Pro-Gly, avec X1, X2 et X3 correspondant à des acides aminés. Comme précisé précédemment, cette séquence hexapeptidique X1-Gly-X2-X3-Pro-Gly est répétée au moins trois fois au sein du peptide selon l'invention. En effet, certains travaux ont montré qu'une répétition de trois fois de ladite séquence entrainait des résultats différents, voire plus intéressants, en termes d'activité de la séquence (Alix 2001).

Selon un mode de réalisation intéressant, le résidu X1 de la partie peptidique A correspond à l'un quelconque des acides aminés. Le résidu X2, quant à lui, correspond avantageusement à un acide aminé choisi parmi Val, Thr, Gln, Ala et Leu. Enfin, le résidu X3 correspond préférentiellement à un acide aminé choisi parmi Ala, Leu et Ile.

Plus préférentiellement encore, X1 et X2 correspondent à Val et X3 à Ala. Ainsi l'hexapeptide correspondant à la partie A du peptide bifonctionnel présente préférentiellement la séquence Val-Gly-Val-Ala-Pro-Gly.

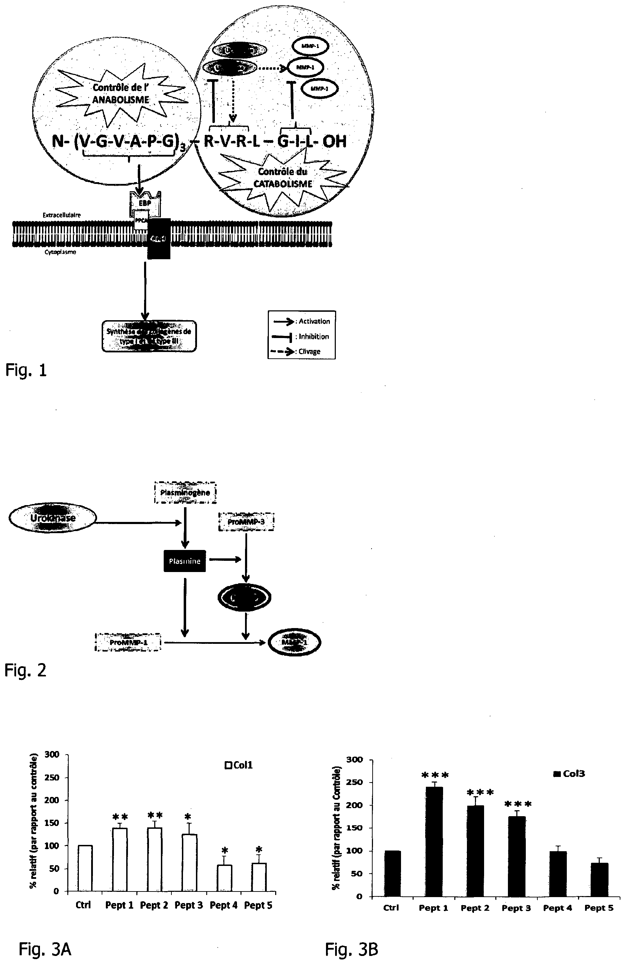

Une telle séquence, répétée au moins trois fois et associée aux parties peptidiques B et C, permet une stimulation significative de l'expression protéique des collagènes, notamment en ce qui concerne le collagène de type III, comme illustré et expliqué sur les figures 3A et 3B annexées et dans l'exemple 1 ci-dessous.

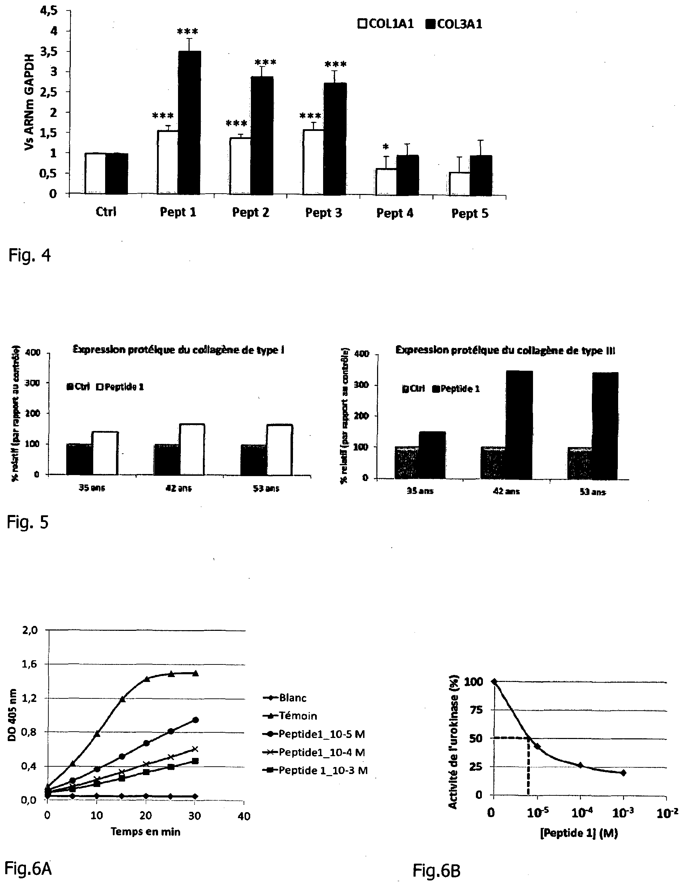

Le peptide selon l'invention permet également une augmentation de l'expression génique des gènes COL1A1 et COL3A1, comme illustré sur la figure 4, codant respectivement pour le collagène de type I et pour le collagène de type III. L'action du peptide bifonctionnel sur la synthèse du collagène type III est particulièrement intéressante ; en effet, ce dernier est connu comme étant le collagène majoritaire au niveau foetal et sa production diminue progressivement pendant l'enfance jusqu'à atteindre un niveau de production très faible chez l'adulte. A titre d'exemple, le collagène type III est 6 fois moins abondant chez l'adulte que le collagène de type I (Melissopoulos, 1998 ; Herbage, 1997). Le collagène de type III est synthétisé majoritairement par les myofibroblastes au cours de la cicatrisation afin de remplacer les tissus lésés. Au niveau foetal, le collagène type III permet une cicatrisation dite « parfaite » (Lorena et al., 2002 ; Ferguson & O'Kane, 2004).

La séquence Val-Gly-Val-Ala-Pro-Gly du peptide bifonctionnel selon l'invention a été conceptualisée à partir des peptides d'élastine ou élastokines dérivant de la dégradation des fibres élastiques. Une telle séquence adopte une conformation en coude β de type VIII du fait de la présence des résidus proline et glycine au sein de ladite séquence X1-Gly-X2-X3-Pro-Gly (Floquet et al., 2004). Ce motif structural est reconnu par une protéine récepteur à l'élastine, traditionnellement citée sous sa dénomination anglophone Elastin Binding Protein, et abrégée EBP. Cette dernière est immobilisée à la surface cellulaire en association avec deux autres sous unités : une Protéine Protectrice ou Cathepsine A (PPCA) et une Neuraminidase (Neu-1), qui sont liées à la membrane cellulaire. L'occupation de la protéine récepteur EBP par la partie peptidique A du peptide bifonctionnel, représenté sur la figure 1, entraine une activation de l'ensemble moléculaire ce qui aboutit notamment à la synthèse de collagène de type I et de type III.

Une telle activation de l'ensemble moléculaire peut également agir sur le chimiotactisme, la prolifération, l'adhésion et la survie des fibroblastes et également sur l'angiogénèse, cette dernière étant essentielle dans le processus de revascularisation des plaies chroniques, telles que les escarres ou les ulcères.

En ce qui concerne la partie peptidique B, celle-ci consiste préférentiellement en un tétrapeptide, c'est-à-dire une séquence comportant 4 acides aminés.

Plus particulièrement, ce tétrapeptide représente un bras pouvant interagir, en tant que substrat ou inhibiteur compétitif, avec une protéase à sérine, l'urokinase. Ainsi, cela permet de libérer les deux autres parties peptidiques A et C, au voisinage de la cellule.

L'urokinase clive préférentiellement une séquence présentant deux acides aminés Arg. De ce fait, la partie peptidique B présente avantageusement la séquence Arg-Y1-Arg-Y2, où Y1 et Y2 correspondent à des acides aminés.

Selon un exemple de réalisation particulier, Y1 et Y2 correspondent chacun à un acide aminé choisi parmi Ser, Tyr, Gly, Ala, Arg, Val et Leu. Ces acides aminés, associés à un site clivable Arg, sont préférentiellement clivés par la protéase urokinase.

Plus préférentiellement encore, la partie peptidique B du peptide bifonctionnel selon l'invention comporte la séquence Arg-Val-Arg-Leu.

La protéase urokinase est l'un des activateurs du système plasminogène-plasmine. Le plasminogène est le substrat physiologique de l'urokinase, qui va permettre de l'activer sous forme de plasmine. Cette activation déclenche une cascade protéolytique qui aboutit notamment à l'activation des MMP-1 et des MMP-3. La cascade enzymatique du système plasminogène/plasmine déclenchée par la protéase urokinase est représentée sur la figure 2 annexée.

Ainsi, l'activité urokinase sur le plasminogène est responsable indirectement de la dégradation du collagène, et notamment du collagène de type I et de type III. De ce fait, la présence de la partie peptidique B dans le peptide bifonctionnel selon l'invention va agir en tant que substrat ou inhibiteur compétitif de l'urokinase. En conséquence, cette dernière clivera les sites Arg présents sur la partie peptidique B et sera moins disponible pour entrainer l'inhibition du plasminogène en plasmine ; la dégradation des molécules de collagène sera donc réduite. L'effet du peptide selon l'invention sur l'activité de l'urokinase est notamment illustré sur les figures 6A et 6B.

En ce qui concerne à présent la partie peptidique C du peptide bifonctionnel, celle-ci consiste avantageusement en un tripeptide, c'est-à-dire une séquence comportant 3 acides aminés.

Préférentiellement, la partie peptidique C présente la séquence Z1-Ile-Z2 avec Z1 et Z2 correspondant chacun à un résidu acide aminé.

Selon un exemple de réalisation intéressant, Z1 est un acide aminé choisi parmi Gly, Ile et Leu tandis que Z2 est choisi parmi Leu, Phe, Ala, Ile, Val.

Plus préférentiellement encore, Z1 correspond à Gly et Z2 correspond à Leu. Ainsi, la partie peptidique C du peptide bifonctionnel présente avantageusement la séquence Gly-Ile-Leu. Le tripeptide Gly-Ile-Leu est en effet susceptible d'occuper une partie du site actif de la MMP-1 et agir en tant qu'inhibiteur compétitif de l'enzyme.

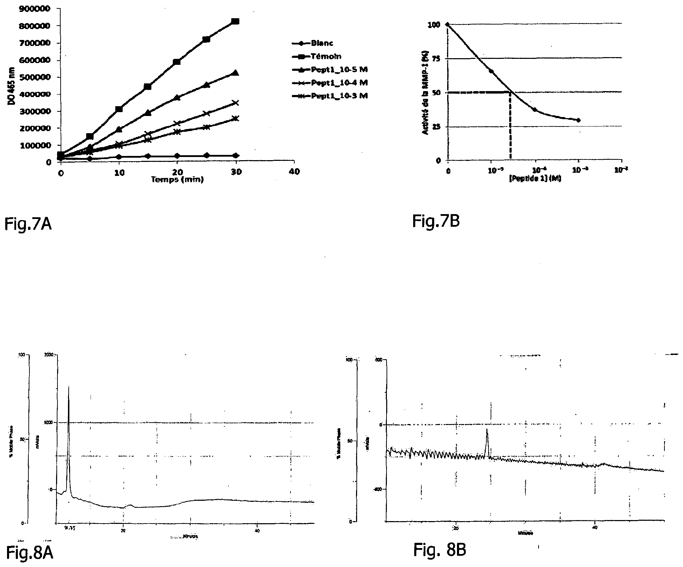

En effet, ces acides aminés présente un intérêt particulier car ils sont susceptibles d'occuper trois des poches de la MMP de type I, les poches P'1, P'2 et P'3. En conséquence, le site actif de la collagénase de type I ne pourra plus chélater les ions Zn²⁺, ces derniers étant essentiels pour l'activité de ladite collagénase. La dégradation des molécules de collagène par les MMP-1 est donc diminuée grâce au peptide bifonctionnel selon l'invention. Cela est en particulier illustré sur les figures 7A et 7B annexées.

Ainsi, selon un mode de réalisation préférentiel, qui permet d'obtenir des résultats intéressants en termes de synthèse de collagène et d'inhibition des MMP, en particulier de la MMP-1, le peptide bifonctionnel selon la présente invention présente la séquence suivante, notée séquence S1, et correspondant à la SEQ ID n°1 :
S1 : N-(Val-Gly-Val-Ala-Pro-Gly)n-Arg-Val-Arg-Leu-Gly-Ile-Leu-OH

Préférentiellement, la séquence Val-Gly-Val-Ala-Pro-Gly est répétée 3 fois ; en d'autres termes, n est avantageusement égal à 3. Cependant, la séquence peut également être répétée un nombre supérieur à 3.

Le peptide selon l'invention peut également présenter, par exemple, les séquences identifiées SEQ ID n°2 à SEQ ID n°26. Cette liste de séquences n'est cependant pas exhaustive et donc non limitative de l'invention.

Notamment, les acides aminés des séquences SEQ ID n°1 à 26 peuvent par exemple être substitués par des acides aminés qui sont chimiquement équivalents, c'est-à-dire présentant des caractéristiques physicochimiques équivalentes ; des substitutions entre acides aminés équivalents se font par exemple entre les acides aminés apolaires aliphatiques Ala, Val, Ile, Leu, ou entre les acides aminés polaires portant un groupement hydroxyle Ser et Thr, entre les acides aminés Asn et Gln, entre les acides aminés portant deux fonctions acide Asp et Glu, etc. Ces informations sont répertoriées dans la base de données des protéases, le Merops (http://merops.sanger.ac.uk/).

Le peptide bifonctionnel selon la présente invention est préférentiellement obtenu par synthèse chimique.

Avantageusement, c'est la technique du fluorénylméthoxycarbonyl (Fmoc)/ter-bytyl (tBu) qui est mise en oeuvre pour obtenir l'élongation des chaines peptidiques, sur des résines Fmoc-Leu-Wang PS.

Cependant, un tel mode de réalisation n'est pas limitatif de l'invention et la synthèse chimique du peptide bifonctionnel peut être obtenue par toute autre technique adaptée à cet effet et connue de l'homme du métier.

Avantageusement, le peptide selon l'invention est lyophilisé de manière à assurer une conservation optimale de ce dernier.

Le peptide bifonctionnel selon la présente invention est tout particulièrement intéressant pour entrer dans la fabrication de compositions cosmétiques destinées à favoriser la réparation et/ou la régénération du tissu dermique. En effet, de par son action sur la production de collagène, ledit peptide permet notamment une limitation de l'apparition de rides.

De plus, il a été montré par les inventeurs que les effets du peptide bifonctionnel selon l'invention sur la production de collagène semblaient dépendre de l'âge des patients. En effet, les résultats montrés dans l'exemple 3 ci-dessous, en relation avec la figure 5, montrent que l'expression du collagène de type I et surtout l'expression du collagène de type III est plus importante chez les patients qui sont plus âgés. De tels résultats confortent donc l'intérêt dudit peptide pour son utilisation dans le cadre de compositions cosmétiques destinées à combattre le vieillissement cutanée.

Cependant, un tel mode de réalisation n'est pas limitatif de l'invention. Par exemple, le peptide bifonctionnel selon l'invention peut également servir à la préparation de compositions pharmaceutiques.

En effet, il s'avère qu'une rupture de l'équilibre entre la synthèse de protéines matricielles, telles que le collagène, et la dégradation de ces mêmes protéines, peut entrainer l'apparition de plaies chroniques, également appelées maladies de cicatrisation chroniques, telles que les escarres, les ulcères ou encore les grands brulés. Ces maladies sont en particulier caractérisées par une surexpression des protéinases matricielles MMPs et sont en général associées à des pathologies telles que le diabète ou les vasculopathies.

Ces maladies de cicatrisation chroniques sont en constante augmentation dans les pays développés, notamment à cause du vieillissement de la population. De ce fait, le traitement et la prise en charge de ces maladies représentent un coût non négligeable.

Au niveau cellulaire, il a été démontré qu'une grande quantité de protéases, et notamment de métalloprotéinases de type MMP-1 était libérée au voisinage de ces plaies chroniques de type escarres. Des études ont également permis de prouver que l'activité de la collagénase de type I, ou MMP-1, augmentait considérablement dans les exsudats de plaies chroniques. Une telle augmentation est responsable de la dégradation des molécules de collagène de type I et de type III qui sont présentes au voisinage de ladite plaie (Barone et al., 1998 ; Shi et al., 2006).

En ce qui concerne à présent plus particulièrement les ulcères, il a également été démontré que de grandes quantités de protéases étaient retrouvées au voisinage de ces plaies. En particulier, des études ont permis d'identifier un taux de MMP-1 65 fois plus élevé que la normale, ces MMPs provoquant alors une dégradation excessive et prolongée des protéines de la matrice extracellulaire et également des facteurs de croissance, entrainant un retard dans la cicatrisation (Fischer et al., 2009).

Enfin, il a été démontré que la concentration en urokinase, activateur du système plasminogène/plasmine comme visible sur la figure 2, et responsable indirectement de l'activation de certaines MMP, était fortement augmentée notamment dans les ulcères des jambes ainsi qu'au niveau des escarres (Werckroth et al., 2004).

Ainsi, il apparait que le peptide selon l'invention, qui permet à la fois une diminution de l'activité des protéases MMP et une augmentation de la synthèse des protéines matricielles, contribuerait à faciliter un traitement de ces plaies chroniques et permettrait de se substituer aux traitements médicaux actuels, qui sont lourds, coûteux et peu efficaces.

Selon un mode de réalisation particulier, le peptide bifonctionnel selon l'invention est intégré dans un patch destiné à être apposé sur la zone du corps qui est touchée par une plaie chronique.

Cependant, un tel mode de réalisation n'est en aucun cas limitatif de l'invention, et il est aisément envisageable d'incorporer le peptide bifonctionnel selon l'invention à une préparation telle qu'une crème, un onguent, un gel etc., pouvant être appliqué sur la plaie à traiter.

Selon un exemple de réalisation, le peptide bifonctionnel est utilisé préférentiellement à une concentration comprise sensiblement entre 10|g/ml et lmg/ml. En effet, aucune toxicité du peptide selon l'invention n'a été constatée sur des fibroblastes dermiques traités avec des concentrations en peptide allant jusqu'à 1mg/ml. De plus, des études menées pour rechercher les gènes de toxicité par l'approche statistique SAM (Signifiance Analysis of Microarrays) ont montré que la mort cellulaire, la désorganisation du tissu conjonctif et l'invasion tumorale ne sont induits qu'à partir d'une forte concentration en peptide bifonctionnel, supérieure à 1mg/ml.

Plus préférentiellement encore, la concentration préconisée pour l'utilisation du peptide, notamment dans les compositions pharmaceutique et/ou cosmétique, est sensiblement égale à 100µg/ml.

D'autres avantages et caractéristiques de l'invention apparaitront également à la lecture des exemples suivants, donnés à titre illustratif et non limitatif.

### Exemple 1 : Effet du peptide bifonctionnel sur la synthèse de collagène - analyse protéique

Les fibroblastes dermiques sont isolés à partir de biopsies de peau d'abdomen obtenues au cours d'opérations chirurgicales pratiquées sur des patients sains âgés de 35 à 75 ans.

Après avoir retiré l'épiderme, les fibroblastes sont récupérés par digestion enzymatique puis sont cultivés à 37°C dans un milieu DMEM (Dubelcco's Modified Eagle Medium) supplémenté par 10% de sérum de veau foetal et par 1% de pénicilline/streptomycine.

Les fibroblastes sont ensuite traités avec le peptide présentant la séquence S1 ou avec différents fragments de cette séquence, à une concentration de 100µg/ml. Les différents fragments testés sont les suivants :
- peptide 1 (pept 1): peptide présentant la séquence S1
- peptide 2 (pept 2): séquence N-(Val-Gly-Val-Ala-Pro-Gly)₃-Arg-Val-Arg-Leu-OH
- peptide 3 (pept 3): N-(Val-Gly-Val-Ala-Pro-Gly)₃-OH
- peptide 4 (pept 4): N-Arg-Val-Arg-Leu-Gly-Ile-Leu-OH
- peptide 5 (pept 5): N-Gly-Ile-Leu-OH

L'expression protéique du collagène de type I et du collagène de type III a ensuite été montrée par Western Blot et quantifiée par ImageJ. Les résultats sont présentés respectivement sur les figure 3A et 3B annexées. Le contrôle (Ctrl) correspond à l'expression de collagène de type I et III au niveau de cellules non traitées par les peptides. Les expériences ont été réalisées en triplicata.

Les résultats visibles sur la figures 3A montrent que le peptide selon l'invention (pept 1) ainsi que les fragments (pept 2 et pept 3) incorporant la séquence Val-Gly-Val-Ala-Pro-Gly qui correspond à la partie A du peptide selon l'invention stimulent de 30% l'expression protéique du collagène de type I. Les fragments peptidiques (pept 4 et pept 5) ne comportant pas la partie A du peptide selon l'invention ne permettent pas la stimulation de la synthèse de collagène de type I ; au contraire, la proportion de collagène synthétisé lorsque les fibroblastes sont en contact avec pept 4 et pept 5 semble plus faible. La séquence Val-Gly-Val-Ala-Pro-Gly est donc bien responsable de la stimulation de la synthèse de collagène de type I.

La figure 3B montre que le peptide selon l'invention présentant la séquence S1 permet une augmentation de la synthèse de collagène de type III ; en effet, comme cela est visible sur l'histogramme, la production de collagène de type III est plus que doublée lorsque les fibroblastes sont traitées avec le peptide présentant la séquence S1.

Il est également intéressant de noter qu'un traitement des fibroblastes avec le peptide présentant la séquence S1 (pept 1) permet une augmentation de la synthèse de collagène de type III de 36% par rapport aux fibroblastes traités par le peptide présentant uniquement la séquence (Val-Gly-Val-Ala-Pro-Gly)₃ (pept 3). Les autres parties du peptide selon l'invention jouent donc également un rôle dans l'augmentation de la synthèse du collagène de type III.

Le peptide selon l'invention est donc tout particulièrement intéressant ; en effet, en plus de permettre une augmentation de la synthèse du collagène de type I, il favorise également la production de collagène de type III.

### Exemple 2 : Effet du peptide bifonctionnel sur la synthèse de collagène - analyse de l'expression génique

L'expression génique des collagènes de type I et III dans les cultures de fibroblastes a également été testée en présence des différents peptides (pept 1 à pept 5) à une concentration de 100µg/ml.

Après 24h de traitement par les différents peptides, les ARNs ont été extraits des cultures à l'aide d'un kit RNeasy® (QIAGEN) puis rétrotranscrits en ADN complémentaires (ADNc) à l'aide du kit RT² First Strand Kit de SABiosciences™.

Des analyses PCR en temps réel sont ensuite effectuées à l'aide du thermocycleur Mx3000p de Stratagene en plaque de 96 puits. Chaque échantillon est analysé en triplicata. Les ADNc servent de matrices dans un mélange réactionnel contenant les réactifs RT² SYBR^{®} Green/ ROX™ qPCR Master Mix (SABiosciences™) et les amorces sens et antisens spécifiques du gène à amplifier. La lecture de la fluorescence est effectuée à la fin de chaque cycle et analysée par le logiciel MxPro (Stratagene). L'expression relative des gènes d'intérêts COL1A1 et COL3A1 codant respectivement pour le collagène de type I et de type III est obtenue en normalisant la quantité de produits d'amplification du gène étudié par la quantité d'ADN amplifié à partir d'un gène « domestique », dans le cas présent l'ARN du gène codant pour la glycéraldéhyde-3-phosphate déshydrogénase (GAPDH). A compter du nombre de cycles correspondant à une amplification possédant une efficacité de 100%, la quantité relative d'ADNc étudié est calculée à l'aide de la méthode du 2-^{ΔΔCT} (Livak & Schmittgen, 2001).

Les résultats, visibles sur la figure 4, confirment ceux obtenus lors de l'étude de l'expression protéique. Le peptide bi-fonctionnel selon l'invention ainsi que les fragments peptidique pept 2 et pept 3 comprenant la séquence (Val-Gly-Val-Ala-Pro-Gly)₃ stimulent l'expression génique des collagènes de type I et III. On constate également une augmentation de la synthèse de collagène de type III lorsque les fibroblastes sont traités par le peptide présentant la séquence S1 (pept 1) par rapport aux cellules mises en présence du peptide 3 contenant la séquence (Val-Gly-Val-Ala-Pro-Gly)₃ seule.

Les peptides dérivés pept 4 et pept 5 contenant la séquence Gly-Ile-Leu n'ont aucun effet sur l'expression des gènes codant pour les collagènes type I et III.

Le contrôle (Ctrl) correspond à l'expression des gènes codant pour les deux types de collagène lorsque les fibroblastes ne sont pas traités.

### Exemple 3 : Effet du peptide bifonctionnel sur la synthèse de collagène - tests de l'expression protéique et génique en fonction de l'âge des patients

Des fibroblastes dermiques provenant de patients plus ou moins âgés (35, 42 et 53 ans) ont été mis en culture et le peptide bifonctionnel selon l'invention présentant la séquence S1 a été testée sur l'expression protéique et génique du collagène de type I et de type III. Les résultats obtenus en ce qui concerne l'expression protéique des deux types de collagène sont visibles sur la figure 5. Il apparait que l'action du peptide selon l'invention sur la synthèse de collagène est plus importante lorsque les fibroblastes sont issus de patients plus âgés.

### Exemple 4 : Inhibition compétitive de l'urokinase par le peptide bifonctionnel

L'action du peptide bifonctionnel selon l'invention sur l'urokinase, une enzyme en amont de la cascade d'activation de la protéinase MMP-1, a été évaluée.

L'activité de l'urokinase sur son substrat synthétique, le D-Glu-Gly-Arg+NHPhNO2 (S2444) a été testée en présence et en absence du peptide selon l'invention à différentes concentrations.

Les résultats sont présentés sur les figures 6A et 6B annexées.

Une cinétique d'hydrolyse de du substrat S2444 (0,3mM) par l'urokinase (9,25*10⁻⁶mM) a été réalisée en présence du peptide bifonctionnel présentant la séquence S1 et concentré à 10⁻³, 10⁻⁴ et 10⁻⁵ M pendant lh. Le témoin correspond à la cinétique en l'absence de peptide. L'activité enzymatique est évaluée par une mesure de l'absorbance (DO) à 405 nm toutes les 5 minutes pendant une durée de 35 min. Les résultats sont visibles sur la figure 6A.

La figure 6B est une représentation graphique de la relation effet/dose entre l'activité de l'urokinase et la concentration de peptide bifonctionnel utilisée au temps 15min. Le trait en pointillé sur la figure permet de déterminer l'IC₅₀, c'est-à-dire la concentration en peptide nécessaire à diminuer de 50% l'activité de l'urokinase. L'IC₅₀ correspond à 0,83*10⁻⁵ M de peptide, soit une concentration massique de peptide de 19µg/ml.

Il apparait donc que le peptide bifonctionnel selon l'invention se comporte comme un inhibiteur compétitif de l'urokinase.

### Exemple 5 : Effet du peptide bifonctionnel sur l'activité de la protéase MMP-1

L'effet du peptide bifonctionnel selon l'invention a ensuite été évalué directement sur l'activité de la MMP-1, qui est une enzyme en aval de la cascade activée par l'urokinase.

Le substrat de la MMP-1 qui a été utilisé pour tester son activité en présence du peptide est un substrat synthétique, le DNP-Pro-Cha-Gly-Cys(Me)-His-Ala-Lys(N-Me-Abz)-NH₂.

Une concentration de 80ng/µl de MMP-1 a été mise en présence de 0,4ng/µl de substrat synthétique, en présence ou non de peptide bifonctionnel concentré à 10⁻³, 10⁻⁴ et 10⁻⁵ M pendant lh. Les résultats sont présentés sur les figure 7A et 7B.

Plus particulièrement, la figure 7A représente une cinétique d'hydrolyse du substrat synthétique de la MMP-1 en présence ou non (témoin) du peptide à différentes concentrations. L'activité enzymatique de la MMP-1 est évaluée par une mesure de l'absorbance (DO) à 465 nm toutes les 5 minutes pendant une durée de 35 min.

La figure 7B est une représentation graphique de la relation dose-effet entre l'activité de la MMP-1 et la concentration de peptide bifonctionnel utilisée au temps 15 min. Le trait en pointillé sur la figure permet de déterminer l'IC₅₀, qui correspond ici à 0,4*10-4 M soit une concentration en peptide bifonctionnel égale à 91µg/ml.

Les effets du peptide selon l'invention ont également été testés sur l'activité de la MMP-1 en présence de son substrat naturel, c'est-à-dire les fibres de collagène de derme humain.

Pour ce faire, la MMP-1 a été préalablement activée par de l'APMA (4-aminophenylmercuric acétate) à 20mM pendant 1h30 à 37°C. La MMP-1 ainsi activée est ensuite déposée sur des coupes de peau de 5µ d'épaisseur en présence ou non du peptide selon l'invention (100µg/ml). Après 3h, les coupes de peau sont immunomarquées afin de révéler la présence de collagène de type I et III puis sont observées en microscopie confocale. Les résultats, non représentés, montrent que, au niveau de la coupe qui n'a pas été traitée par le peptide, les collagènes I et III ont été totalement dégradés par la MMP-1. Au contraire, en présence du peptide bifonctionnel, l'action de ladite MMP-1 est inhibée ; en effet, les collagènes I et III sont détectés.

Le peptide bifonctionnel selon l'invention inhibe la dégradation des collagènes en inhibant *in* situ l'activité des MMP-1.

### Exemple 6 : Clivage du peptide bifonctionnel par l'urokinase

Au niveau péricellulaire on observe un excès d'urokinase, de l'ordre de 10⁻⁴ M. Le peptide peut donc être clivé par l'urokinase, cette dernière agissant en particulier au niveau des résidus arginine (Arg) de la partie B du peptide selon l'invention.

Des études ont été menées par HPLC sur colonne Chromolith Jupiter C18 (50 x 4,6 mm) et en appliquant un gradient linéaire de 0,1% de TFA acétonitrile dans 0,1 % de TFA eau, de 0 à 100% pendant 100 min. Les résultats montrent la présence d'un pic spectral à 11,75 min caractéristique du peptide bi-fonctionnel (Figure 8A). Cependant, après action d'un excès d'urokinase sur le peptide bi-fonctionnel (0,45 mM), le pic spectral est différent ; en effet, le nouveau pic est élué à 20 min caractérisant ainsi un clivage du peptide bi-fonctionnel par l'urokinase (figure 8B).

Ainsi, dans des conditions de vieillissement cutané, en présence de quantités importantes d'urokinase, le peptide bifonctionnel selon l'invention est clivé, libérant ainsi la partie C-terminale dudit peptide incorporant la séquence Gly-Ile-Leu.

### Exemple 7 : Effets de la séquence Gly-Ile-Leu sur l'activité de la MMP-1

La séquence Gly-Ile-Leu a été choisie par les inventeurs pour sa capacité à occuper les poches P'1, P'2 et P'3 de la collagénase de type I, la MMP-1. L'activité de cette dernière a été testée sur son substrat synthétique, le DNP-Pro-Cha-Gly-Cys(Me)-His-Ala-Lys(N-Me-Abz)-NH₂, en présence ou en absence du peptide 5 présentant la séquence Gly-Ile-Leu aux concentration 10⁻⁵, 10⁻⁴ et 10⁻³ M.

Les résultats (non représentés) montrent que le peptide 5 diminue de façon dose dépendante l'activité de la MMP-1 sur son substrat synthétique. L'IC 50 du peptide Gly-Ile-Leu a été mesurée ; elle est de 0,71*10-4 M soit une concentration de peptide de 21,3µg/ml.

La partie C terminale Gly-Ile-Leu du peptide bifonctionnel selon l'invention peut donc être générée en cas d'excès d'urokinase et entrainer une inhibition de l'activité de la MMP-1. Ces données confirment l'intérêt du peptide pour lutter contre le vieillissement cutané et/ou la cicatrisation de plaies chroniques.

Des études ont également été menées sur un modèle d'étude plus complet : les modèles de peau *ex vivo* permettant de mimer le vieillissement cutané ainsi que certaines pathologies associées. Les résultats obtenus ne sont pas illustrés mais ils confirment les résultats obtenus *in vitro.*

Bien entendu, l'invention n'est pas limitée aux exemples illustrés et décrits précédemment qui peuvent présenter des variantes et modifications sans pour autant sortir du cadre de l'invention.

### BIBLIOGRAPHIE

- Alix, A.J. A turning point in the knowledge of the structure-function-activity relations of elastin. J Soc Biol 2001; 195(2):181-93
- Barone, E.J., et al., Interleukin-1alpha and collagenase activity are elevated in chronic wounds. Plast Reconstr Surg, 1998 102(4): p. 1023-7; discussion 1028-9.
- Ferguson, M.W. and S. O'Kane, Scar-free healing: from embryonic mechanisms to adult therapeutic intervention. Philos Trans R Soc Lond B Biol Sci, 2004 359(1445): p. 839- 50.
- Fisher, G.J., et al., Collagen fragmentation promotes oxidative stress and elevates matrix metalloproteinase-1 in fibroblasts in aged human skin. Am J Pathol, 2009 174(1): p. 101-14.
- Floquet, N., Héry-Huynh, S., Dauchez, M., Derreumaux, P., Tamburro, A.M., Alix, A.J. Structural characterization of VGVAPG, an elastin-derived peptide. Biopolymers, 2004; 76(3):266-80. Review
- Herbage, D. Collagènes et protéoglycannes du derme: données actuelles. in Biologie de la peau. 1997
- Hornebeck, W., Inflamm-âge et cascades protéolytiques. Médecine et longévité. Vol. 1. 2009: Elsevier Masson. 38-43
- Livak, K.J. and T.D. Schmittgen, Analysis of relative gene expression data using real-time quantitative PCR and the 2(-Delta Delta C(T)) Method.Methods, 2001 25(4): p. 402-8.
- Lorena, D., et al., Normal scarring: importance of myofibroblasts. Wound Repair Regen, 2002 10(2): p. 86-92.
- Mélissopoulos A., L.C., La peau. Médicales et internationales ed. 1998.
- Shi, B., et al., Effect of vacuum assisted closure on collagenase activity in human chronic wound. Zhonghua Zheng Xing Wai Ke Za Zhi, 2006 22(6): p. 465-7.
- Voorhees, J. J., et al, Collagen degradation in aged/photodamaged skin in vivo and after exposure to matrix metalloproteinase-1 in vitro. J Invest Dermatol, 2003 ; 120(5) : p. 842-8
- Weckroth, M., et al., Epithelial tissue-type plasminogen activator expression, unlike that of urokinase, its receptor, and plasminogen activator inhibitor-1, is increased in chronic venous ulcers. Br J Dermatol, 2004 151(6): p. 1189-96

## Revendications

1. Peptide bifonctionnel apte à activer la synthèse de collagène et à inhiber la production de métallo-protéinases matricielles, **caractérisé en ce qu'**il présente une séquence comportant trois parties peptidiques A, B et C :
- la première partie peptidique A, correspondant à un hexapeptide répété au moins trois fois, ladite partie A étant apte à se lier à une protéine récepteur liant l'élastine pour stimuler la synthèse de collagène, et présentant la séquence X1-Gly-X2-X3-Pro-Gly avec :
o X1 correspondant à un acide aminé quelconque,
o X2 correspondant à un acide aminé choisi parmi Val, Thr, Gln, Ala, Leu,
o X3 correspondant à un acide aminé choisi parmi Ala, Leu, Ile,
- la deuxième partie peptidique B, correspondant à un tétrapeptide apte à agir en tant qu'inhibiteur compétitif de la protéase urokinase et à être clivé par ladite protéase, et présentant la séquence Arg-Y1-Arg-Y2, avec Y1 et Y2 correspondant chacun à un acide aminé choisi parmi Ser, Tyr, Gly, Ala, Arg, Val, Leu, et,
- la troisième partie peptidique C, correspondant à un tripeptide occupant au moins un site actif des métallo-protéinases matricielles pour permettre une inhibition desdites protéinases, et présentant la séquence Z1-Ile-Z2, avec :
o Z1 correspondant à un acide aminé choisi parmi Gly, Ile, Leu et
o Z2 correspondant à un acide aminé choisi parmi Leu, Phe, Ala, Ile, Val.

2. Le peptide bifonctionnel selon la revendication 1 **caractérisé en ce que** la première partie peptidique A dudit peptide présente la séquence Val-Gly-Val-Ala-Pro-Gly.

3. Le peptide bifonctionnel selon la revendication 1 ou 2 **caractérisé en ce que** la deuxième partie peptidique B dudit peptide présente la séquence Arg-Val-Arg-Leu.

4. Le peptide bifonctionnel selon l'une quelconque des revendications 1 à 3 **caractérisé en ce que** la troisième partie peptidique C dudit peptide présente la séquence Gly-Ile-Leu.

5. Le peptide bifonctionnel selon l'une quelconque des revendications 1 à 4 **caractérisé en ce qu'**il présente la séquence S1 suivante, correspondant à la séquence SEQ ID n°1 :
S1: N-(Val-Gly-Val-Ala-Pro-Gly)n-Arg-Val-Arg-Leu-Gly-Ile-Leu-OH
où N et OH correspondent respectivement aux extrémités N-terminale et C-terminale dudit peptide
et où n=3

6. Le peptide bifonctionnel selon l'une quelconque des revendications 1 à 4 **caractérisé en ce qu'**il correspond à l'une des séquences SEQ ID n°2 à SEQ ID n°26.

7. Le peptide bifonctionnel selon l'une quelconque des revendications 1 à 6 **caractérisé en ce qu'**il est obtenu par synthèse chimique.

8. Le peptide bifonctionnel selon l'une quelconque des revendications 1 à 7 **caractérisé en ce qu'**il est conservé sous forme lyophilisée.

9. Le peptide bifonctionnel selon l'une quelconque des revendications 1 à 8 pour utilisation pour le traitement des maladies de cicatrisation chroniques.

10. Le peptide bifonctionnel pour utilisation selon la revendication 9 pour le traitement des escarres ou des ulcères.

11. Le peptide bifonctionnel selon l'une quelconque des revendications 1 à 8 pour utilisation pour la réparation et/ou la régénération du tissu dermique.

12. Le peptide bifonctionnel pour utilisation selon la revendication 11 pour le traitement du vieillissement cutané.

13. Composition cosmétique et/ou pharmaceutique incorporant le peptide bifonctionnel selon l'une quelconque des revendications 1 à 8.

14. La composition cosmétique et/ou pharmaceutique selon la revendication 13 dans laquelle la concentration en peptide bifonctionnel est comprise entre 10µg/mL et 1mg/mL, et de préférence sensiblement égale à 100µg/mL.

## Patentansprüche

1. Bifunktionelles Peptid, das in der Lage ist, die Synthese von Kollagen zu aktivieren und die Produktion von Matrix-Metallo-Proteinasen zu hemmen, **dadurch gekennzeichnet, dass** es eine Sequenz aufweist, die drei Peptidteile A, B und C umfasst:
- den ersten Peptidteil A, der einem mindestens dreimal wiederholten Hexapeptid entspricht, wobei der besagte Teil A in der Lage ist, sich an ein Rezeptor-Protein zu binden, das Elastin bindet, um die Synthese von Kollagen zu stimulieren, und die Sequenz X1-Gly-X2-X3-Pro-Gly aufweist, wobei:
• X1 einer beliebigen Aminosäure entspricht,
• X2 einer aus Val, Thr, Gln, Ala, Leu ausgewählten Aminosäure entspricht,
• X3 einer aus Ala, Leu, Ile ausgewählten entspricht,
- den zweiten Peptidteil B, der einem Tetrapeptid entspricht, das als kompetitiver Inhibitor der Protease Urokinase wirkt und durch die besagte Protease gespalten werden kann, und die Sequenz Arg-Y1-Arg-Y2 aufweist, wobei Y1 und Y2 jeweils einer aus Ser, Tyr, Gly, Ala, Arg, Val, Leu ausgewählten Aminosäure entsprechen, und
- den dritten Peptidteil C, der einem Tripeptid entspricht, das mindestens ein aktives Zentrum der Matrix-Metallo-Proteinasen einnimmt, um eine Hemmung der besagten Proteinasen zu ermöglichen, und die Sequenz Z1-Ile-Z2 aufweist, wobei:
o Z1 einer aus Gly, Ile, Leu ausgewählten Aminosäure entspricht, und
o Z2 einer aus Leu, Phe, Ala, Ile, Val ausgewählten Aminosäure entspricht.

2. Bifunktionelles Peptid nach Anspruch 1, **dadurch gekennzeichnet, dass** der erste Peptidteil A des besagten Peptids die Sequenz Val-Gly-Val-Ala-Pro-Gly aufweist.

3. Bifunktionelles Peptid nach Anspruch 1 oder 2, **dadurch gekennzeichnet, dass** der zweite Peptidteil B des besagten Peptids die Sequenz Arg-Val-Arg-Leu aufweist.

4. Bifunktionelles Peptid nach irgendeinem der Ansprüche 1 bis 3, **dadurch gekennzeichnet, dass** der dritte Peptidteil C des besagten Peptids die Sequenz Gly-Ile-Leu aufweist.

5. Bifunktionelles Peptid nach irgendeinem der Ansprüche 1 bis 4, **dadurch gekennzeichnet, dass** es die folgende S1-Sequenz aufweist, die der Sequenz SEQ ID n°1 entspricht:
S1: N-(Val-Gly-Val-Ala-Pro-Gly)n-Arg-Val-Arg-Leu-Gly-Ile-Leu-OH
wo N und OH jeweils den N-terminal- und C-terminal-Enden des besagten Peptids entsprechen,
und wo n=3.

6. Bifunktionelles Peptid nach irgendeinem der Ansprüche 1 bis 4, **dadurch gekennzeichnet, dass** es einer der Sequenzen SEQ ID n°2 bis SEQ ID n°26 entspricht.

7. Bifunktionelles Peptid nach irgendeinem der Ansprüche 1 bis 6, **dadurch gekennzeichnet, dass** es durch chemische Synthese erhalten wird.

8. Bifunktionelles Peptid nach irgendeinem der Ansprüche 1 bis 7, **dadurch gekennzeichnet, dass** es in lyophilisierter Form gelagert wird.

9. Bifunktionelles Peptid nach irgendeinem der Ansprüche 1 bis 8 zur Verwendung für die Behandlung von heilenden chronischen Erkrankungen.

10. Bifunktionelles Peptid zur Verwendung nach Anspruch 9 zur Behandlung von Druckgeschwüren oder Geschwüren.

11. Bifunktionelles Peptid nach irgendeinem der Ansprüche 1 bis 8 zur Verwendung für die Reparatur und/oder Regeneration des Hautgewebes.

12. Bifunktionelles Peptid zur Verwendung nach Anspruch 11 für die Behandlung von Hautalterung.

13. Kosmetische und/oder pharmazeutische Zusammensetzung, die das bifunktionelle Peptid nach irgendeinem der Ansprüche 1 bis 8 enthält.

14. Kosmetische und/oder pharmazeutische Zusammensetzung nach Anspruch 13, bei der die bifunktionelle Peptidkonzentration zwischen 10µg/mL und 1mg/mL liegt, und vorzugsweise im Wesentlichen gleich 100µg/mL ist.

## Claims

1. A bifunctional peptide capable of activating the synthesis of collagen and inhibiting the production of matrix metallo-proteinases, wherein it has a sequence including three peptide portions A, B and C:
- the first peptide portion A, corresponding to an at least three times repeated hexapeptide, said portion being capable of binding to a receptor protein binding elastin in order to stimulate the synthesis of collagen, and having the sequence X1-Gly-X2-X3-Pro-Gly with:
• X1 corresponding to any amino acid,
• X2 corresponding to an amino acid selected from Val, Thr, Gln, Ala, Leu,
• X3 corresponding to an amino acid selected from Ala, Leu, Ile,
- the second peptide portion B, corresponding to a tetrapeptide capable of acting as a competitive inhibitor of the protease urokinase and of being cleaved by said protease, and having the sequence Arg-Y1-Arg-Y2, with Y1 and Y2 each corresponding to an amino acid selected from Ser, Tyr, Gly, Ala, Arg, Val, Leu, and,
- the third peptide portion C corresponding to a tripeptide occupying at least one active site of the matrix metallo-proteinases in order to permit an inhibition of said proteinases, and having the sequence Z1-Ile-Z2, with:
• Z1 corresponding to an amino acid selected from Gly, Ile, Leu, and
• Z2 corresponding to an amino acid selected from Leu, Phe, Ala, Ile, Val.

2. The bifunctional peptide according to claim 1, wherein the first peptide portion A of said peptide has the sequence Val-Gly-Val-Ala-Pro-Gly.

3. The bifunctional peptide according to claim 1 or 2, wherein the second peptide portion B of said peptide has the sequence Arg-Val-Arg-Leu.

4. The bifunctional peptide according to any one of claims 1 to 3, wherein the third peptide portion C of said peptide has the sequence Gly-Ile-Leu.

5. The bifunctional peptide according to any one of claims 1 to 4, wherein it has the following S1sequence, corresponding to the sequence SEQ ID n°1:
S1: N-(Val-Gly-Val-Ala-Pro-Gly)n-Arg-Val-Arg-Leu-Gly-Ile-Leu-OH
wherein N and OH respectively correspond to the N-terminal and C-terminal ends of said peptide,
and wherein n=3.

6. The bifunctional peptide according to any one of claims 1 to 4, wherein it corresponds to one of the sequences SEQ ID n°2 to SEQ ID n°26.

7. The bifunctional peptide according to any one of claims 1 to 6, wherein it is obtained by chemical synthesis.

8. The bifunctional peptide according to any one of claims 1 to 7, wherein it is preserved in lyophilized form.

9. The bifunctional peptide according to any one of claims 1 to 8 for use for the treatment of the chronic scarring diseases.

10. The bifunctional peptide for use according to claim 9 for the treatment of the pressure sores or the ulcers.

11. The bifunctional peptide according to any one of claims 1 to 8 for use for the repair and/or regeneration of the dermal tissue.

12. The bifunctional peptide for use according to claim 11 for the treatment of skin aging.

13. A cosmetic and/or pharmaceutical composition incorporating the bifunctional peptide according to any one of claims 1 to 8.

14. The cosmetic and/or pharmaceutical composition according to claim 13, wherein the concentration of bifunctional peptide varies between 10µg/mL and 1mg/mL, and is preferably substantially equal to 100µg/mL.
